# EUROPEAN PATENT APPLICATION

(11) **EP 4 660 288 A1**
(43) Date of publication of application: **10.12.2025**
(21) Application number: 24180773.4
(22) Date of filing: 07.06.2024
(51) Int. Cl.: C12M 1/00, C12M 1/06, B01F 35/41, C12M 1/12, C12M 1/02

(54) **BIOREACTOR, BIOREACTOR VESSEL AND CLOSURE COMPONENT FOR A BIOREACTOR VESSEL**

(71) Applicant: Sartorius Stedim Biotech GmbH, 37079 Göttingen (DE)
(72) Inventor: PULLIN, Jeremy, Stonehouse, GL10 3UT (GB); RADDATZ, Lukas, 37079 Göttingen (DE); BENDALL, Richard, Stonehouse, GL10 3UT (GB); CHRISTIE, Alexander, Stonehouse, GL10 3UT (GB); GABBITAS, Tim, Stonehouse, GL10 3UT (GB); ZHU, Zicheng, Stonehouse, GL10 3UT (GB); HANCOX, Neil, Stonehouse, GL10 3UT (GB)
(74) Representative: Mewburn Ellis LLP

(57) **Abstract**

A closure component is provided for closing an upper opening of a rigid-walled bioreactor vessel, in use the vessel containing a biological medium and the upper opening providing access to the interior of the vessel. The component has: a lid having a mounting for fixing to a rim of the upper opening such that the lid closes the upper opening; a shaft which extends downwards from the lid; and one or more impellers mounted to the shaft. The mounting forms a bearing with the rim of the opening when fixed thereto. The lid, the shaft and the one or more impellers are integrally formed such that the component is a one-piece assembly in which the lid, the shaft and the one or more impellers are rotatable together as a single unit to stir the contents of the vessel.

## Description

### Field of the Invention

The present invention relates to a bioreactor, a bioreactor vessel and a closure component for a bioreactor vessel.

### Background

Cell cultivation is the performance of cell growth or fermentation in a favourable artificial environment. Example applications of cell cultivation are for the production of mAbs, vaccines, or recombinant proteins, and cell and gene therapies.

Cell line selection and early process development for cell cultivation is often performed using parallel cultivations in small scale, benchtop bioreactor vessels, such as the Ambr 15^{™} microbioreactor system from Sartorius AG which enables 24 or 48 parallel cultivations with disposable bioreactor vessels holding volumes of cell culture solution in the range from 10 to 15 mL. Promising cell lines and processes identified from these microbioreactor systems can be further developed by parallel cultivations in higher throughput and higher volume bioreactor vessels, an example being the Ambr 250^{™} bioreactor system from Sartorius which performs high throughput cell cultivation with 12 or 24 disposable bioreactors for volumes of cell culture solution in the range from 100 to 250 mL.

The best candidates from these developments can ultimately be transferred to larger scale bioreactor vessels such as the Univessel^{™} SU bioreactor from Sartorius which allows working volumes of from 0.6 L to 2 L and the Biostat Cplus^{™} sterilizable-in-place bioreactor also from Sartorius which allows working volumes of from 5 L to 30 L. This streamlined approach to development allows many variables to be explored, while promoting cost-effective experimentation by making efficient use of labour, facility space, capital, media and consumables.

Focusing on the Univessel^{™} SU bioreactor, this has a single-use, polycarbonate vessel and accommodates a large number of sensing and control interfaces. For example, vessel ports located on the lid vessel include three addition ports, three ports with dip tubes for harvesting or media addition, three sensor ports, a thermowell for inserting a temperature sensor and a needle-free septum port for sampling. The vessel also has a stirrer shaft descending from the lid and featuring two 3-blade segment impellers. Aeration takes place either in a submerged configuration via an L-type sparger with small holes and/or through the headspace.

Relatively large scale bioreactor vessels such as the Univessel^{™} SU are complex devices, assembled from many individual components. Operators need to be properly trained on correct assembly and operation procedures for these devices. In addition, the number of components and the need to provide a reliable product leads to extended product development stages, which mitigates against making changes to design features. It would be desirable to be able to provide a bioreactor vessel which is less complex to assemble and which can be more flexibility adapted to the needs of specific users. It would be desirable to be able to provide a bioreactor vessel which provides improved cell cultivation performance.

Bioreactor vessels can also be used to perform in vitro transcription or translation for cell-free production of nucleic acids (e.g. RNA or DNA) or proteins.

WO 2020/002598 A1 proposes a bioreactor for RNA in vitro transcription in which the inner surface of the reaction vessel has an ellipsoid or an oval inner geometry.

EP 3728550 A1 proposes a lid configuration for a bioreactor in which the lid is provided with one or more 3D-printed sensor or controller elements.

The present invention has been devised in light of the above considerations.

### Summary of the Invention

In general terms, the present invention provides a rigid-walled bioreactor vessel for containing a biological medium. The vessel may have an internal volume of at least 10 mL, and preferably 50 mL, 100 mL, 500 mL or 2 L. The vessel may have an internal volume of at most 50 L, and preferably 10 L or 5 L.

The wall of the vessel may have an inner surface which is substantially ovoid, ellipsoid or spheroid in shape. In this way, the vessel can avoid having any corner regions (such as are found around the bottom edge of a convention cylindrical vessel) where biological medium can be trapped or held up, reducing mixing efficiency. However, this does not exclude that the inner surface can take other shapes, such as a cylindrical shape.

Conveniently, the wall of the vessel may be formed by an additive manufacture (AM) process. This can allow the vessel to be more easily tailored to accommodate different cell growth or fermentation requirements, e.g. by adding or subtracting sensing or control interfaces. Also, by using AM each feature of the vessel can be easily re-labelled as needed, making it easier and quicker for an operator to set up the product, e.g. by reducing an operator's dependence on an instruction manual. In addition, by using AM it is possible to add a unique identifier to every vessel, improving product traceability.

One or more heat exchange channels for exchanging heat with contents of the vessel may be integrated into the wall of the vessel such that the material of the wall forms the one or more channels, the or each channel having a respective inlet configured to receive a flow of a heat exchange fluid into the channel, and a respective outlet configured to discharge the flow of the heat exchange fluid from the channel. For example, the or each heat exchange channel can wind helically around the vessel. As the material of the wall forms the one or more channels, when the wall is formed by AM, this process also forms the one or more channels.

In a first aspect, the present invention provides a closure component for closing an upper opening of a rigid-walled bioreactor vessel, in use the vessel containing a biological medium and the upper opening providing access to the interior of the vessel, wherein the component has:
a lid having a mounting for fixing to a rim of the upper opening such that the lid closes the upper opening;
a shaft which extends downwards from the lid; and
one or more impellers mounted to the shaft;
wherein the mounting forms a bearing with the rim of the opening when fixed thereto, and the lid, the shaft and the one or more impellers are integrally formed such that the component is a one-piece assembly in which the lid, the shaft and the one or more impellers are rotatable together as a single unit to stir the contents of the vessel.

Advantageously, by providing a lid which is also rotatable with the shaft, the total number of components involved in the vessel can be reduced. Also, the bearings for the shaft can be located at a greater radial distance from the shaft than might otherwise be the case. This radial distance improves stability and in particular can reduce the tendency of the shaft to yaw about its axis. With this improved stability, it is possible to avoid supporting the bottom end of the shaft on a locating pin at the base of the vessel - such locating pins and their interface to a shaft being a location in conventional vessels where cells can be trapped and waste products can build up.

The closure component (i.e. at least the lid, the shaft and the impellers) can be formed by an additive manufacture (AM) process.

The ratio of the total length of extension of the shaft downwards from the lid to the diameter of the bearing may be 4 or less, and preferably may be 3 or less. Having the total length of extension limited in this way also helps to reduce the tendency of the shaft to yaw.

Preferably, with increasing distance downwards from the lid, the diameter of the shaft first increases and then decreases. In this way the shaft can be locally stiffened and/or strengthened only where such stiffening/strengthening is needed. For example, the shaft may carry a first impeller (e.g. a toroidal impeller) at its bottom end and a second impeller (e.g. a Rushton impeller) at a location between the bottom and the lid. With such an arrangement, with bending forces on the shaft typically at their greatest at or near to the second impeller, local thickening of the shaft in this region can help to resist those forces.

Conveniently, the bearing may be a rolling bearing containing a circumferential row of rolling elements (e.g. ball bearings), the rim and the mounting forming opposite halves of a track for the rolling elements when the mounting is fixed to the rim. Such bearings can combine low rolling resistance with durability and reliability. Preferably, the track is configured such that it is fillable with and retains a sealing liquid (such as isopropyl alcohol, IPA) when the mounting is fixed to the rim, such that the bearing forms an airtight seal. Thus effectively the track can provide a circumferentially continuous reservoir for the sealing liquid which not only lubricates the rolling elements but also forms a sterile a barrier between the external environment and the contents of the vessel.

Typically the component further has an engagement formation (for example a splined projection) on the top side of the lid for engaging with a motor drive. The component can thus be rotated by engaging the motor drive to the formation.

As the lid rotates in use with the shaft and the one or more impellers, typically the lid is free of any ports, e.g. of the type which in lids of conventional bioreactor vessels may be used for adding, harvesting, sensing etc. Thus the lid can provide a continuous and unbroken surface from its mounting which forms a bearing with the rim of the upper opening of the vessel to the shaft.

In a second aspect, the present invention provides a bioreactor including a rigid-walled bioreactor vessel (e.g. the rigid-walled bioreactor vessel described in general terms above) for containing a biological medium, the vessel having an upper opening providing access to the interior of the vessel, and bioreactor further including the closure component of the first aspect for closing the upper opening and for stirring contents of the vessel.

The bottom end of the shaft may be spaced from the facing, bottom inner surface of the wall of the vessel such that the bottom end of the shaft is unsupported by the vessel. As explained above, the improved shaft stability provided by the closure component advantageously makes it possible to avoid supporting the bottom end of the shaft on a locating pin at the base of the vessel.

The vessel may include a sparger that is configured to deliver gas bubbles to the contents of the vessel through bubble-forming openings of the sparger, the openings being located directly below the bottom end of the shaft. Thus, as the bottom end of the shaft does not have to be supported at the base of the vessel, this location can be advantageously occupied instead by the sparger.

The vessel may include an external gas supply port configured to connect to a gas supply outside the vessel and the sparger may be a foraminous member containing fora which extend through the member from an upstream side in fluid communication with the gas supply port to a downstream surface at which the fora terminate in the bubble-forming openings, the foraminous member being embedded within the wall of the vessel such that the downstream surface forms a single smooth surface with the inner surface of the wall of the vessel.

Indeed, more generally, in a third aspect, the present invention provides a rigid-walled bioreactor vessel (e.g. the rigid-walled bioreactor vessel described in general terms above) for containing a biological medium, the vessel including a sparger that is configured to deliver gas bubbles (e.g. from a location at the base of the vessel) to the contents of the vessel, the sparger including an external gas supply port configured to connect to a gas supply outside the vessel, and further including a foraminous member containing fora which extend through the member from an upstream side in fluid communication with the gas supply port to a downstream surface at which the fora terminate in bubble-forming openings, the foraminous member being embedded within the wall of the vessel such that the downstream surface forms a single smooth surface with the inner surface of the wall of the vessel.

The single smooth surface can thus help the sparger to avoid forming a trap for cells where waste products can build up.

Conveniently, the embedded sparger of the second or third aspect may be formed by AM as the wall of the vessel is itself being formed by AM.

The sparger of the second or third aspect may further include a supply passage linking the gas supply port to the upstream side of the foraminous member, the gas supply port being located on the exterior surface of the vessel at a spacing from the foraminous member, and the supply passage being integrated with and extending parallel to the wall of the vessel between the gas supply port and the foraminous member such that the material of the wall also forms the supply passage. Thus, conveniently, the supply passage may be formed by AM as the wall of the vessel is itself being formed by AM.

In the sparger of the second or third aspect having the embedded foraminous member, the fora may be provided by interconnected porosity of the foraminous member.

The rigid-walled bioreactor vessel of the second or third aspect may further include one or more heat exchange channels for exchanging heat with contents of the vessel, the or each channel being integrated into the wall of the vessel such that the material of the wall also forms the one or more channels, and the or each channel having a respective inlet configured to receive a flow of a heat exchange fluid into the channel, and a respective outlet configured to discharge the flow of the heat exchange fluid from the channel.

Indeed, more generally, in a fourth aspect, the present invention provides a rigid-walled bioreactor vessel (e.g. the rigid-walled bioreactor vessel described in general terms above) for containing a biological medium and including one or more heat exchange channels for exchanging heat with contents of the vessel, the or each channel being integrated into the wall of the vessel such that the material of the wall also forms the one or more channels, and the or each channel having a respective inlet configured to receive a flow of a heat exchange fluid into the channel, and a respective outlet configured to discharge the flow of the heat exchange fluid from the channel.

In the vessel of any one of the second to fourth aspects, the or each heat exchange channel may wind helically around the vessel.

Conveniently, when the wall of the vessel of any one of the second to fourth aspects is formed by an AM process, the or each heat exchange channel may be formed as part of that process.

The or each heat exchange channel may have a circular, D-shaped or tear-shaped (pointed end upwards) cross-section. A tear-shaped cross-section can be more convenient to form by AM than a circular cross-section. A D-shaped cross-section can have heat transfer benefits, as discussed further below.

The vessel of any one of the second to fourth aspects preferably includes plural of the heat exchange channels. One or more of the channels can thus be used to warm contents of the vessel, while simultaneously one or more other of the channels can be used to cool contents of the vessel.

For example, in one approach when the vessel of any one of the second to fourth aspects includes plural of the heat exchange channels, one of the heat exchange channels may be a warming channel contained in the wall of a lower portion of the vessel, and a second one of the heat exchange channels may be a cooling channel contained in the wall of an upper portion of the vessel. The warming and cooling channels are thus useable such that a flow of first heat exchange fluid in the warming channel warms liquid contents of the vessel, and a flow of second heat exchange fluid in the cooling channel condenses vapour forming above a top surface of the liquid contents of the vessel. When the channels are helically wound around the vessel, the spacing between adjacent turns of the cooling channel may be less than the spacing between adjacent turns of the warming channel. In this way, the cooling channel turns can have a higher packing density to better condense the vapour, and reflecting that the inner surface of the vessel will generally have a lower heat transfer coefficient with a gas than a liquid. In a development of the approach, a third one of the heat exchange channels may be a second cooling channel contained in the wall of the lower portion of the vessel, the second cooling channel being useable such that a flow of a third heat exchange fluid in the second cooling channel cools liquid contents of the vessel. The second cooling channel in combination with the warming channel enables improved temperature control of the liquid contents of the vessel, e.g. enabling more rapid achievement of target temperatures while helping to avoid or reduce under or over-shoots.

Indeed, in a variant approach when the vessel of any one of the second to fourth aspects includes plural of the heat exchange channels, one of the heat exchange channels may be a warming channel contained in the wall of at least a lower portion of the vessel, and a second one of the heat exchange channels may be a cooling channel contained in the wall of at least a lower portion of the vessel. The warming and cooling channels are then useable such that a flow of first heat exchange fluid in the warming channel warms liquid contents of the vessel, and a flow of second heat exchange fluid in the cooling channel cools liquid contents of the vessel. Thus the variant approach also enables improved temperature control of the liquid contents of the vessel.

In the rigid-walled bioreactor vessel of any one of the second to fourth aspects, each heat exchange channel may be configured to favour heat transfer between the heat exchange fluid flowing in the channel and the contents of the vessel over heat transfer between the heat exchange fluid flowing in the channel and exterior of the vessel. For example, each channel may adopt any one, any two or all three of the following configuration options.

According to one configuration option, the amount of the material of the wall forming each heat exchange channel is less on the side of the channel facing the inner surface of the vessel than on the side of the channel facing the exterior of the vessel. In particular, on a cross-section through each heat exchange channel the average thickness of the material of the wall forming the channel may be less (e.g. at least 50% less) on the 90° quadrant of the channel cross-section facing the inner surface of the vessel than on the 90° quadrant of the channel cross-section facing the exterior of the vessel.

According to another configuration option, the material of the wall forming each heat exchange channel may protrude into the interior of the vessel to increase the amount of surface for heat transfer between the wall of the vessel at the channel and the contents of the vessel and/or to promote turbulent mixing of the contents of the vessel at the inner surface of the vessel. When each channel is helically wound around the vessel, the protrusion of adjacent turns of the channel creates a recess therebetween on the inner surface of the vessel. On a cross-section through the adjacent turns of the channel, the depth of this recess may be at least 50% of the internal diameter of the channel cross-section as measured across the wall of the vessel.

According to yet another configuration option, the cross-section of each heat exchange channel may be shaped to favour heat transfer between the fluid flowing in the channel and the material of the wall forming the channel on the side of the channel facing the inner surface of the vessel than the side of the channel facing the exterior of the vessel. For example, each channel may have a D-shaped cross-section, with the curved side of the D facing the interior of the vessel.

In a fifth aspect, the present invention provides a method of forming the rigid-walled bioreactor vessel of the third or fourth aspect, the method including:
using an additive manufacture process to form the bioreactor vessel.

Thus one example of the fifth aspect provides a method of forming a rigid-walled bioreactor vessel for containing a biological medium, the vessel including a sparger that is configured to deliver gas bubbles to the contents of the vessel, the sparger having an external gas supply port configured to connect to a gas supply outside the vessel and a foraminous member containing fora which extend through the member from an upstream side in fluid communication with the gas supply port to a downstream surface at which the fora terminate in bubble-forming openings, the foraminous member being embedded within the wall of the vessel such that the downstream surface forms a single smooth surface with the inner surface of the wall of the vessel, the method including:
using an additive manufacture process to form the bioreactor vessel.

Another example of the fifth aspect provides a method of forming a rigid-walled bioreactor vessel for containing a biological medium and including one or more heat exchange channels for exchanging heat with contents of the vessel, the or each channel being integrated into the wall of the vessel such that the material of the wall also forms the one or more channels, and the or each channel having a respective inlet configured to receive a flow of a heat exchange fluid into the channel, and a respective outlet configured to discharge the flow of the heat exchange fluid from the channel, the method including:
using an additive manufacture process to form the bioreactor vessel.

In a sixth aspect, the present invention provides a method of forming the closure component of the first aspect, the method including:
using an additive manufacture process to form the closure component.

Thus an example of the sixth aspect provides a method of forming a closure component for closing an upper opening of a rigid-walled bioreactor vessel, in use the vessel containing a biological medium and the upper opening providing access to the interior of the vessel, wherein the component has: a lid having a mounting for fixing to a rim of the upper opening such that the lid closes the upper opening; a shaft which extends downwards from the lid; and one or more impellers mounted to the shaft; wherein the mounting forms a bearing with the rim of the opening when fixed thereto, the method including:
using an additive manufacture process to integrally form the lid, the shaft and the one or more impellers as a one-piece closure component, whereby the lid, the shaft and the one or more impellers are rotatable together as a single unit to stir the contents of the vessel.

In a seventh aspect, the present invention provides a method of forming the bioreactor of the second aspect, the method including:
using an additive manufacture process to form the bioreactor vessel, and
using a further additive manufacture process to form the closure component.

Thus an example of the seventh aspect provides a method of forming a bioreactor including a rigid-walled bioreactor vessel for containing a biological medium, the vessel having an upper opening providing access to the interior of the vessel, and the bioreactor further including a closure component for closing the upper opening, wherein the component has: a lid having a mounting for fixing to a rim of the upper opening such that the lid closes the upper opening; a shaft which extends downwards from the lid; and one or more impellers mounted to the shaft; wherein the mounting forms a bearing with the rim of the opening when fixed thereto, the method including:
using an additive manufacture process to form the bioreactor vessel, and
using a further additive manufacture process to integrally form the lid, the shaft and the one or more impellers as a one-piece closure component, whereby the lid, the shaft and the one or more impellers are rotatable together as a single unit to stir the contents of the vessel.

The invention includes the combination of the aspects and features described except where such a combination is clearly impermissible or expressly avoided.

### Summary of the Figures

Embodiments and experiments illustrating the principles of the invention will now be discussed with reference to the accompanying figures in which:
**Figure 1** shows perspective views of a bioreactor vessel and a closure component for that vessel with the closure component lifted off an upper opening the vessel to provide access to the interior of the vessel;
**Figure 2** shows a first side view of the bioreactor vessel and the closure component with the closure component closing the upper opening of the vessel;
**Figure 3** shows a perspective cut away view of the bioreactor vessel;
**Figure 4A** shows in perspective side view of the closure component;
**Figure 4B** shows a longitudinal cross-sectional view of the closure component along plane B-B indicated in Figure 4A;
**Figure 5** is a detailed view of the region Z indicated in Figure 4B;
**Figure 6A** shows a second side view of the bioreactor vessel (from the opposite side of the vessel relative to the first side view of Figure 2);
**Figure 6B** shows a third side view of the bioreactor vessel (from a position midway between the viewpoints of Figure 2 and Figure 6A);
**Figure 7A** shows a longitudinal cross-sectional view of the bioreactor vessel along plane C-C indicated in Figure 6A;
**Figure 7B** shows a longitudinal cross-sectional view of the bioreactor vessel along plane D-D indicated in Figure 6B;
**Figure 8** is a detailed view of the region Y indicated in Figure 7B; and
**Figure 9** is the same view as Figure 8 for a variant of the vessel;
**Figure 10** shows a view of a variant of the vessel on a cross-section corresponding to the longitudinal cross-sectional view of Figure 7B;
**Figure 11** is a detailed view of the region X indicated in Figure 10;
**Figure 12** is a top view of the vessel of Figure 10 looking down through its upper opening; and
**Figure 13** is an SEM micrograph of a porous, sintered ceramic member.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

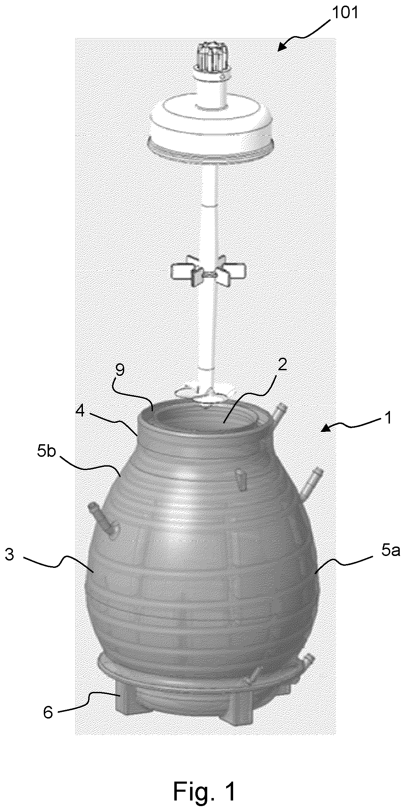

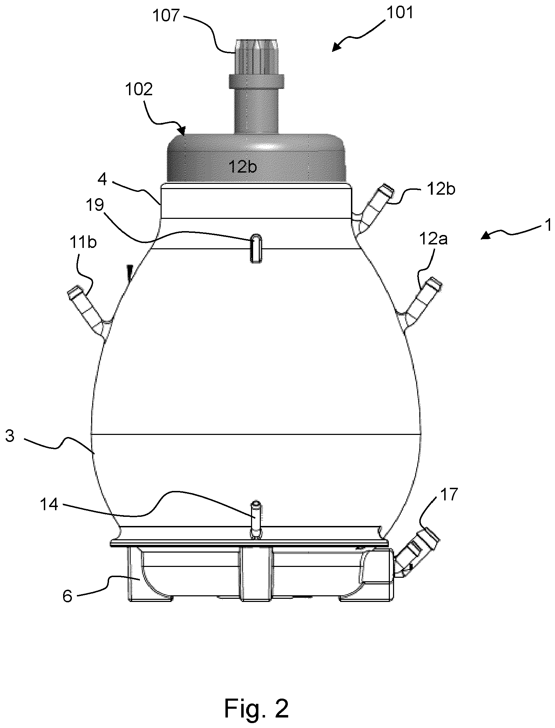

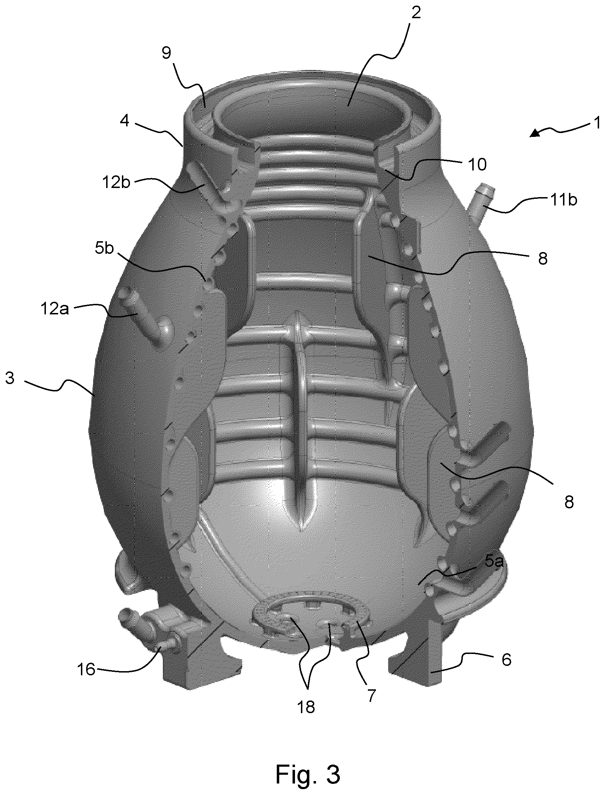

Figure 1 shows perspective views of a bioreactor vessel 1 and a closure component 101 for that vessel with the closure component lifted off an upper opening 2 of the vessel to provide access to the interior of the vessel; Figure 2 shows a first side view of the bioreactor vessel and the closure component with the closure component closing the upper opening of the vessel; and Figure 3 shows a perspective cut away view of the bioreactor vessel.

The vessel 1 has a rigid main wall 3 which provides a substantially ovoid inner surface orientated such that accesses the interior of the vessel through the upper opening 2 is at a short neck region 4 at the narrow end of the ovoid. In use the vessel contains a liquid biological medium with a gas space in the top of the vessel above a top surface of the medium. The vessel can be sized for a range of internal volumes, i.e. from as little as 10 mL and up to 50 L.

Two heat exchange channels 5a, 5b for exchanging heat with contents of the vessel wind helically around the vessel 1, these channels being integrated into the wall 3. A first one of the heat exchange channels 5a is a warming channel contained in the wall of a lower portion of the vessel, in use the warming channel containing a flow of a first heat exchange fluid (e.g. hot water) for warming the biological medium to a controlled temperature. A second one of the heat exchange channels 5b is a cooling channel contained in the wall of an upper portion of the vessel, in use the cooling channel containing a flow of a second heat exchange fluid (e.g. cold water) for condensing vapour forming above the top surface of the biological medium.

The vessel sits on a base 6, which lifts the bottom of the vessel slightly off the ground. At the bottom of the interior of the vessel a sparger 7 is provided for introducing gas bubbles into the biological medium.

Circumferential rows of spaced baffles 8 project radially inwardly from the inner surface of the vessel 1. The baffles disrupt the rotational flow (discussed below) of the biological medium, thereby preventing the formation of a central vortex that could otherwise lead to inefficient mixing and cell damage from high shear conditions in such a vortex. The improved mixing provided by baffles helps to ensure that nutrients, gases and cells are evenly distributed throughout the biological medium, which enhances cell growth and productivity. It also improves the transfer of gases into the biological medium from the upper gas space. This is important in aerobic fermentation processes, where oxygen supply is critical for cell metabolism. In addition, the improved mixing helps to distribute heat evenly throughout the vessel, ensuring that the temperature is uniform and consistent, which is also important for optimising growth conditions.

The vessel 1 can conveniently be formed as a one-piece component by one or more AM techniques, e.g. SLA (stereolithography) can be used to form the wall 3 with its integrated heat exchange channels 5a, 5b from suitable 3D printer resin, such as Somos^{™} BioClear^{™} available from Stratsys, Inc. An advantage of using AM is that it facilitates the development and production of complex geometries, which can then be further developed and adapted as needed.

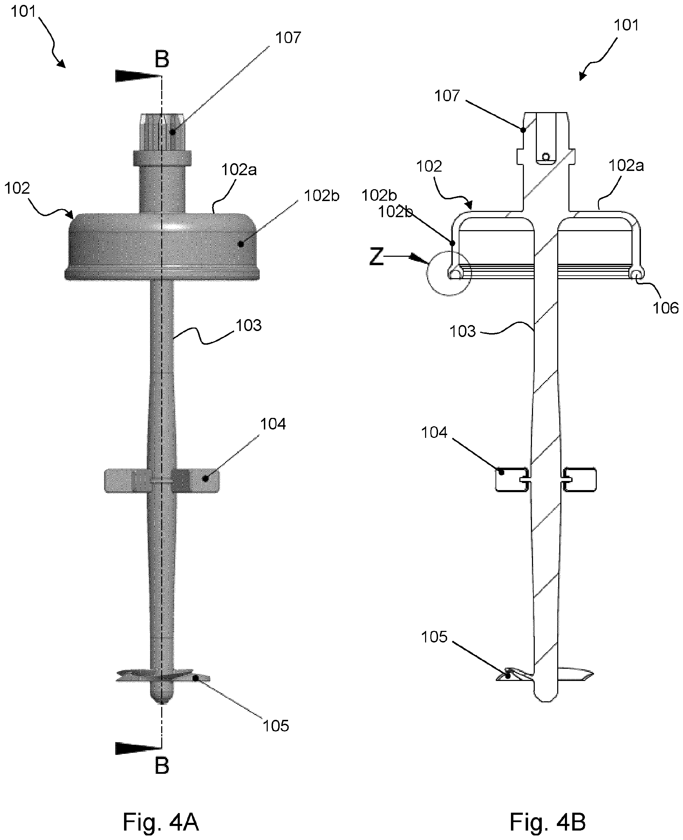

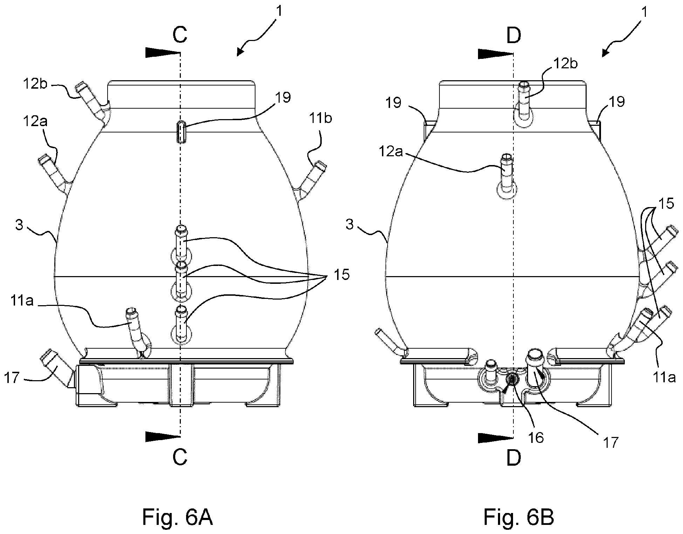

The features of the vessel 1 are discussed in more detail below in relation to Figures 6 to 8. However, we focus next on the closure component 101, which Figure 4A shows in perspective side view, and which Figure 4B shows a longitudinal cross-sectional view along plane B-B indicated in Figure 4A.

The closure component 101 has a lid 102 with top portion 102a and a cylindrical side wall 102b that extends downwards from the top portion 102a to sit within with an annular recess 9 formed in the rim around the upper opening 2 the vessel. Extending upwards from the centre of the lid 102 is a splined mount 107 for engagement with a complementary output drive of a rotary motor. Extending downwards from the centre of the lid 102 is a shaft 103 which carries a Rushton impeller 104 at just over the midpoint of the shaft and a toroidal impeller 105 at the bottom end of the shaft, the impellers being used to produce a rotational flow in the biological medium. The shaft is sized such that it extends vertically almost the entire length of the vessel but with its bottom end spaced from the bottom inner surface of the vessel 1.

The closure component 101 is a one-piece assembly in which the lid 102, the shaft 103 and the impellers 104, 105 are integrally formed so that, when driven by the motor, not just the shaft and impellers but the entire assembly rotates as a single unit. Conveniently, the closure component can also be formed by AM, facilitating the development and production of complex geometries. In this way, the number, position and type (for example, Rushton, toroidal, elephant ear) of impellers can be changed as needed. For example, the closure component can be formed from polyamide material using SLS (Selective Laser Sintering).

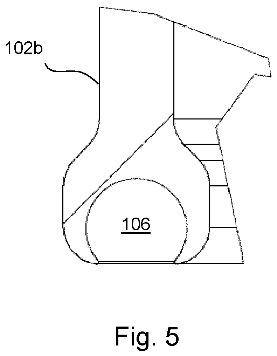

Figure 5 is a detailed view of the region Z indicated in Figure 4B. At the bottom circular edge of the cylindrical side wall 102b is a mounting in the form of an annular groove 106 into which a circumferential row of ball bearings (not shown) can be snap-fittingly loaded such that the bearings are retained in the groove even when the closure component 101 is orientated with the open side of the groove facing downwards. To fit the closure component to the vessel 1, the shaft 103 is inserted through the upper opening 2 of the vessel and the lid 102 lowered onto the upper opening until the bottom circular edge of the cylindrical side wall 102b is received in the annular recess 9 around the rim of the upper opening. The ball bearings loaded in the annular groove 106 locate between a pair of concentric raised retaining ridges formed in the base of the annular recess 9, whereby the annular groove and the annulus 10 in the base of the recess between these ridges together form a circular track for the bearings to rotate in. A suitable sealing liquid such as IPA can then be poured into recess 9, which thus forms a circumferentially continuous reservoir for the sealing liquid. This liquid not only lubricates the ball bearings, but also reduces noise and forms a sterile and airtight barrier between the external environment and the contents of the vessel as the closure component 101 thus rotates on the vessel 1.

Advantageously, as the lid 102 rotates with the shaft 103, the total number of components involved in the vessel can be reduced compared to a conventional bioreactor in which a stirring shaft penetrates through and rotates within the confines of a stationary vessel lid. Moreover, as the bearing is located at the radially outer edge of the lid, rather than close to the shaft, it counteracts any tendency of the shaft to yaw about its rotational axis by providing effective, reactive, anti-yaw torque. This improved rotational stability of the shaft is a reason why it is possible for the bottom end of the shaft to be spaced from the bottom inner surface of the vessel, thereby avoiding a need for a locating pin extending upwards from the base of the vessel to engage and stabilise the shaft. To enhance this stability, the total length of extension of the shaft 103 downwards from the lid 102 to the diameter of the bearing may be 4 or less, and preferably 3 or less.

Further improvements in the stability of the shaft 103 can provided by increasing the diameter of the shaft and then decreasing its diameter with increasing distance downwards from the lid 102. Local stiffening and/or strengthening of the shaft can thus be focused on locations where bending forces on the shaft are at their greatest. In particular, the shaft diameter can be at its greatest in the midpoint region of the Rushton impeller 104.

As the lid 102 rotates with the shaft 103, it is free of any ports for purposes of adding, harvesting, sensing etc. Instead, these interfaces are formed in the wall 3 of the vessel 1, to which we now return to discuss in more detail.

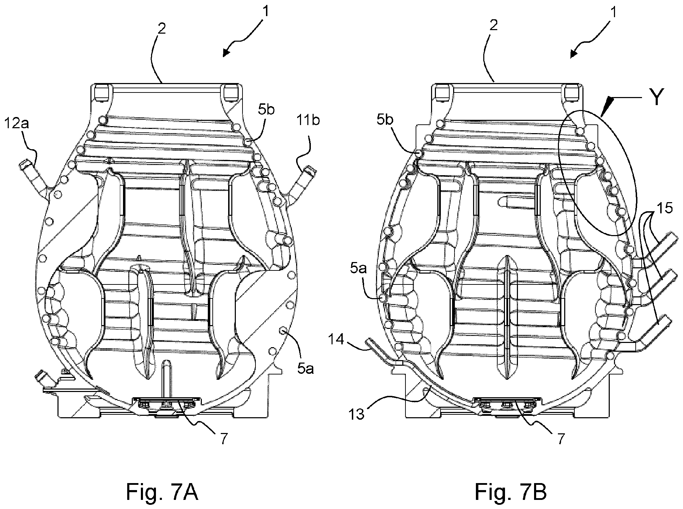

Figure 6A shows a second side view of the bioreactor vessel (from the opposite side of the vessel relative to the first side view of Figure 2); Figure 6B shows a third side view of the bioreactor vessel (from a position midway between the viewpoints of Figure 2 and Figure 6A); Figure 7A shows a longitudinal cross-sectional view of the bioreactor vessel along plane C-C indicated in Figure 6A; and Figure 7B shows a longitudinal cross-sectional view of the bioreactor vessel along plane D-D indicated in Figure 6B.

At dead zones where mixing is poor in the biological medium, waste products which can be toxic to cells and inhibit their growth may accumulate. However, the ovoid inner surface of the vessel 1 helps to reduce or eliminate such dead zones. The ovoid shape thus improves the distribution of nutrients, gases, and cells, which can promote cell growth and productivity in the biological medium. The ovoid shape is also stronger than conventional cylindrical vessel shapes. This increases vessel stability, reducing effects of vibration and rocking when high motor speeds are used to rotate the closure component 101.

The heat exchange channels 5a, 5b which wind helically around the vessel 1 are integrated into the wall 3 of the vessel and have respective inlets 11a, 11b and respective outlets 12a, 12b extending outwards from the wall with hose bard fittings for receiving and discharging flows of suitable heat exchange fluids into the channels. The integration of the channels into the wall rather than the use of conventional heating and cooling jackets helps to increase heat transfer rates and therefore improves the thermal responsivity of the bioreactor. The spacing between adjacent turns of the upper cooling heat exchange channel 5b is less than the spacing between adjacent turns of the lower warming channel 5a to produce a higher packing density of the turns of the cooling channel 5b. This assists the condensation of vapour in the gas space above the biological medium.

Although not shown in the drawings, a variant of the vessel may have a further helically wound heat exchange channel in the wall of the lower portion of the vessel 1 with a respective inlet and outlet for carrying in use a flow of a further heat exchange fluid for cooling the biological medium. By having the ability to both warm (via the warming heat exchange channel 5a) and cool (via the further heat exchange channel) the biological medium, improved temperature control of the biological medium can be achieved.

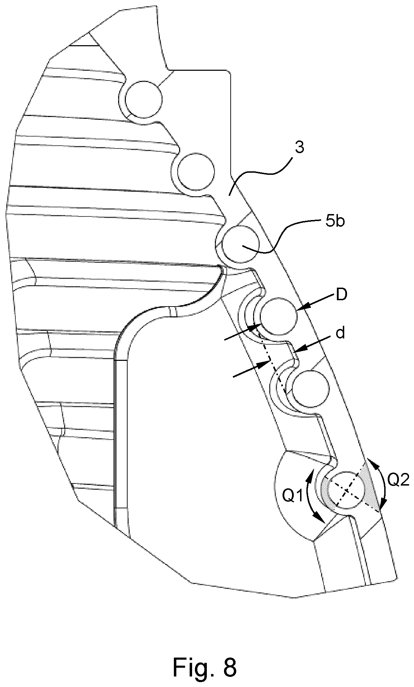

The channel 5a, 5b are configured to favour heat transfer between the heat exchange fluids flowing in the channels and the contents of the vessel over heat transfer between the heat exchange fluids and the exterior of the vessel by virtue of the material of the wall forming each heat exchange channel protruding into the interior of the vessel, as best shown in Figures 3, 7A and 7B. Figure 8 is a detailed view of the region Y indicated in Figure 7B, and shows that the protrusion of adjacent turns of each channel creates a recess therebetween on the inner surface of the vessel. On a cross-section through the adjacent turns, such as shown in Figure 8, the depth d of this recess is at least 50% of the internal diameter D of the channel cross-section as measured across the wall of the vessel.

As well as their protrusion into the interior of the vessel, the heat exchange channels 5a, 5b a further feature which favours heat transfer between the heat exchange fluids and the biological medium in the vessel 1 is the relative distribution of the material of the wall forming each channel. In particular, there is significantly less wall material on the side of the channel facing the inner surface of the vessel than on the side of the channel facing the exterior of the vessel, and thus there is a reduced barrier to heat transfer from that material on the interior-facing side of the channel. For example, on the cross-section of Figure 8, the average thickness of the material of the wall 3 forming the channel 5b is significantly less (e.g. at least 50% less) on the 90° quadrant Q1 of the cross-section facing the inner surface of the vessel than on the 90° quadrant Q2 of the cross-section facing the exterior of the vessel.

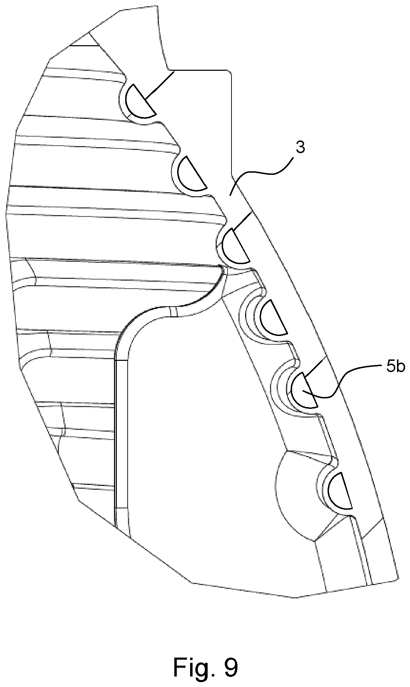

In a variant of the vessel, shown in Figure 9, the shape of the cross-sectional shape of each heat exchange channel 5a, 5b is yet a further feature which favours heat transfer between the heat exchange fluids and the biological medium. In the variant, the cross-section, rather than being circular, is D-shaped with the curved side of the D facing the interior of the vessel.

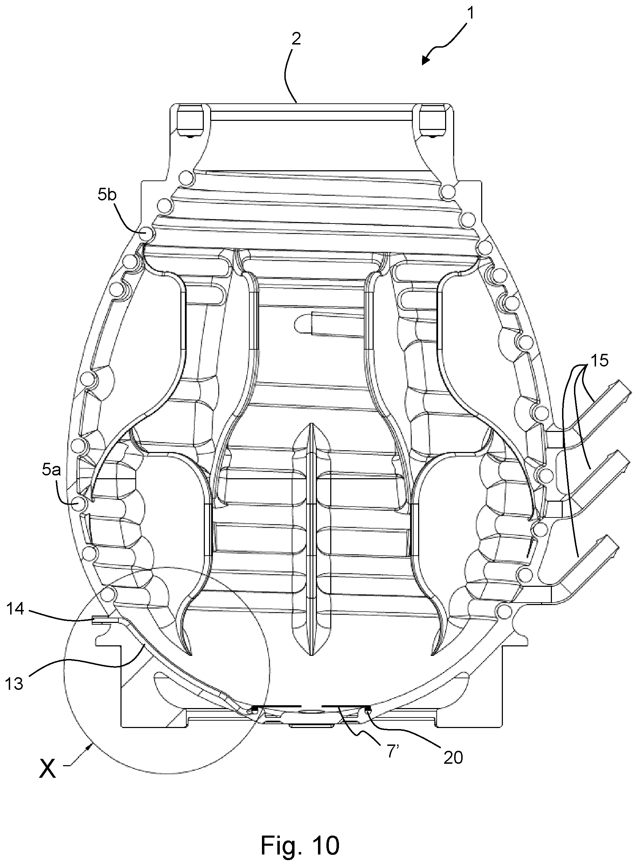

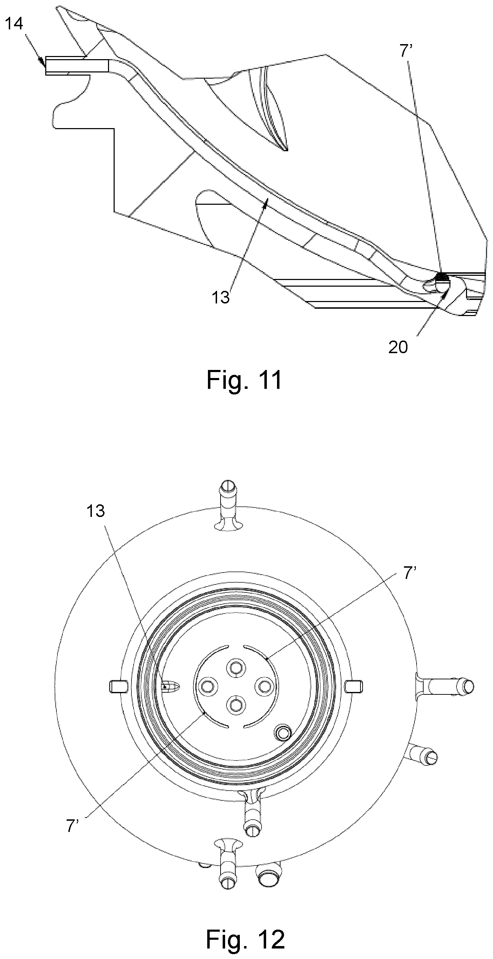

The sparger 7 includes a ring manifold 7 that sits on short legs just above the bottom of vessel 1. The ring manifold contains concentric circumferential rows of bubble-forming openings which are fed gas by a supply passage 13 connecting the ring manifold to an external gas supply port 14, the supply passage being integrated with and extending parallel to the wall 3 of the vessel. In a variant of the vessel, however, this ring manifold can be replaced by a foraminous member 7' which is embedded within the wall 3 of the vessel 1. The member may be formed of ceramic or polymer, and its fora may be provided, for example, by interconnected porosity of the member. An upstream side of the member receives gas from the supply passage 13 and at a downstream surface of the member the fora terminate in the bubble-forming openings. Advantageously, this downstream surface can then form a single smooth surface with the inner surface of the wall of the vessel, whereby a potential trap for cells and waste product can be avoided. Figure 10 shows a view of such a bioreactor vessel corresponding to the longitudinal cross-sectional view of Figure 7B; Figure 11 is a detailed view of the region X indicated in Figure 10; and Figure 12 is a top view of the vessel of Figure 10 looking down through the upper opening 2. The foraminous member 7' is in two halves, each forming a nearly complete semicircle and with both halves being fed gas at their undersides by an underlying ring manifold 20 at the end of the supply passage 13.

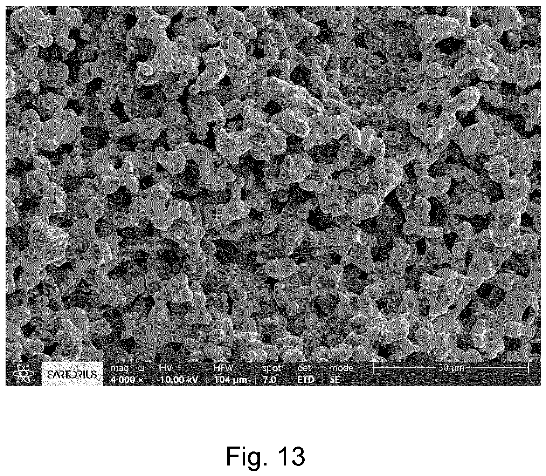

When the foraminous member 7' is made of polymer, it can conveniently be formed using SLS, the volume energy density in the SLS process being controlled at a lower level than a typical SLS process for producing fully dense parts such as the closure component 101. This lower energy process results in reduced coalescence of polymer powder particles, typically leaving pores of approximately 10-15µm between sintered particles. These pores are interconnected and homogeneously distributed across the member, allowing gas to escape across the entire upper surface of the member. Advantageously, the SLS process can be integrated with other AM processes for forming the vessel 1. When the foraminous member 7' is made of ceramic, it can be formed by a process in which initially a slurry of mixed ceramic particles and porogens at an appropriate ratio is cured by UV light in a Digital Light Processing (DLP) process. This provides a "green" part, which is then subject to two consecutive post-processing stages in a furnace. The first stage is debinding at 600°C for 20-22 hours to burn away the polymeric binder and the porogens. The debound part then undergoes the second stage which is sintering at 1400°C - 1700°C, depending on the desired porosity level, for 2 hours. The sintering temperature can control the bulk porosity and individual pore sizes (e.g. from 1µm to 5µm), and is generally lower than temperatures that are used for forming fully dense parts. When the vessel 1 is formed by AM, the ceramic member 7' can be introduced into the vessel at a suitable semi-built stage and fully incorporated as further vessel material is added around it. Figure 13 is an SEM micrograph of the ceramic member.

As well as the inlets 11a, 11b, outlets 12a, 12b and gas supply port 14, the wall 3 of the vessel can contain a number of other ports or interfaces to the contents of vessel such as:
- Sampling ports 15 for sampling the biological medium,
- A perfusion port 16,
- A harvesting port 17, and
- pH patches 18

At the top of the vessel lugs 19 can be provided for attaching to mechanical supports extending from the motor.

When the vessel 1 is formed by AM, the number and positions of ports and interfaces can be changed as needed. Different port connectors, such as tri clamps or multiplexes can be introduced. Other sensor options can be introduced such as for performing conductivity, temperature or Raman spectroscopy measurements. The number, positions and shapes of the baffles 8 can be altered. The number, routes and cross-sections of the heat exchange channels 5a, 5b can be altered. For example, circular and D-shaped cross-section channels are shown in Figures 7A to 9. However, another option is to adopt teardrop shaped cross-section channels (pointed end of the teardrop uppermost), such a shape being readily formable by AM.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A closure component (101) for closing an upper opening (2) of a rigid-walled bioreactor vessel (1), in use the vessel containing a biological medium and the upper opening providing access to the interior of the vessel, wherein the component has:
a lid (102) having a mounting for fixing to a rim of the upper opening such that the lid closes the upper opening;
a shaft (103) which extends downwards from the lid; and
one or more impellers (104, 105) mounted to the shaft;
wherein the mounting forms a bearing with the rim of the opening when fixed thereto, and the lid, the shaft and the one or more impellers are integrally formed such that the component is a one-piece assembly in which the lid, the shaft and the one or more impellers are rotatable together as a single unit to stir the contents of the vessel.

2. The closure component according to claim 1, wherein the ratio of the total length of extension of the shaft downwards from the lid to the diameter of the bearing is 4 or less, and preferably is 3 or less.

3. The closure component according to claim 1 or 2, wherein with increasing distance downwards from the lid, the diameter of the shaft first increases and then decreases.

4. The closure component according to any one of the previous claims, wherein the bearing is a rolling bearing containing a circumferential row of rolling elements, the rim and the mounting forming opposite halves of a track (9, 10) for the rolling elements when the mounting is fixed to the rim.

5. The closure component according to claim 4, wherein the track is configured such that it is fillable with and retains a sealing liquid when the mounting is fixed to the rim, such that the bearing forms an airtight seal.

6. A bioreactor including a rigid-walled bioreactor vessel (1) having an upper opening (2) providing access to the interior of the vessel, and the closure component (101) of any one of the previous claims for closing the upper opening and for stirring contents of the vessel.

7. The bioreactor according to claim 6, wherein the bottom end of the shaft (103) is spaced from the facing, bottom inner surface of the wall (3) of the vessel such that the bottom end of the shaft is unsupported by the vessel.

8. The bioreactor according to claim 6 or 7, wherein the vessel includes a sparger that is configured to deliver gas bubbles to the contents of the vessel through bubble-forming openings of the sparger, the openings being located directly below the bottom end of the shaft.

9. The bioreactor according to claim 8, wherein the sparger includes an external gas supply port (14) configured to connect to a gas supply outside the vessel and further includes a foraminous member (7') containing fora which extend through the member from an upstream side in fluid communication with the gas supply port to a downstream surface at which the fora terminate in the bubble-forming openings, the foraminous member being embedded within the wall of the vessel such that the downstream surface forms a single smooth surface with the inner surface of the wall of the vessel.

10. A rigid-walled bioreactor vessel (1) for containing a biological medium, the vessel including a sparger that is configured to deliver gas bubbles to the contents of the vessel, the sparger including an external gas supply port (14) configured to connect to a gas supply outside the vessel, and further including a foraminous member containing fora which extend through the member from an upstream side in fluid communication with the gas supply port to a downstream surface at which the fora terminate in bubble-forming openings, the foraminous member being embedded within the wall (3) of the vessel such that the downstream surface forms a single smooth surface with the inner surface of the wall of the vessel.

11. The bioreactor vessel according to claim 10, wherein the sparger further includes a supply passage (13) linking the gas supply port to the upstream side of the foraminous member, the gas supply port being located on the exterior surface of the vessel at a spacing from the foraminous member, and the supply passage being integrated with and extending parallel to the wall of the vessel between the gas supply port and the foraminous member such that the material of the wall also forms the supply passage.

12. The bioreactor vessel according to claim 10 or 11 further including one or more heat exchange channels (5a, 5b) for exchanging heat with contents of the vessel, the or each channel being integrated into the wall of the vessel such that the material of the wall also forms the one or more channels, and the or each channel having a respective inlet (11a, 11b) configured to receive a flow of a heat exchange fluid into the channel, and a respective outlet (12a, 12b) configured to discharge the flow of the heat exchange fluid from the channel.

13. A method of forming a rigid-walled bioreactor vessel (1) for containing a biological medium, the vessel including a sparger that is configured to deliver gas bubbles to the contents of the vessel, the sparger having an external gas supply port (14) configured to connect to a gas supply outside the vessel and a foraminous member (7') containing fora which extend through the member from an upstream side in fluid communication with the gas supply port to a downstream surface at which the fora terminate in bubble-forming openings, the foraminous member being embedded within the wall (3) of the vessel such that the downstream surface forms a single smooth surface with the inner surface of the wall of the vessel, the method including:
using an additive manufacture process to form the bioreactor vessel.

14. A method of forming a closure component (101) for closing an upper opening (2) of a rigid-walled bioreactor vessel (1), in use the vessel containing a biological medium and the upper opening providing access to the interior of the vessel, wherein the component has: a lid (102) having a mounting for fixing to a rim of the upper opening such that the lid closes the upper opening; a shaft (103) which extends downwards from the lid; and one or more impellers (104, 105) mounted to the shaft; wherein the mounting forms a bearing with the rim of the opening when fixed thereto, the method including:
using an additive manufacture process to integrally form the lid, the shaft and the one or more impellers as a one-piece closure component, whereby the lid, the shaft and the one or more impellers are rotatable together as a single unit to stir the contents of the vessel.

15. A method of forming a bioreactor including a rigid-walled bioreactor vessel (1) for containing a biological medium, the vessel having an upper opening (2) providing access to the interior of the vessel, and the bioreactor further including a closure component (101) for closing the upper opening, wherein the component has: a lid (102) having a mounting for fixing to a rim of the upper opening such that the lid closes the upper opening; a shaft (103) which extends downwards from the lid; and one or more impellers (104, 105) mounted to the shaft; wherein the mounting forms a bearing with the rim of the opening when fixed thereto, the method including:
using an additive manufacture process to form the bioreactor vessel, and
using a further additive manufacture process to integrally form the lid, the shaft and the one or more impellers as a one-piece closure component, whereby the lid, the shaft and the one or more impellers are rotatable together as a single unit to stir the contents of the vessel.
